# FASCICULE DE BREVET EUROPEEN

(11) **EP 0 575 603 B1**
(45) Date de publication et mention de la délivrance du brevet: **23.10.1996**
(21) Numéro de dépôt: 93904075.4
(22) Date de dépôt: 13.01.1993
(51) Int. Cl.: A61K 7/00, A61K 7/13, C09C 3/08, C09B 67/08, C09B 63/00

(54) **PRODUIT A BASE DE PARTICULES MINERALES COLOREES COMPORTANT UN PIGMENT MELANIQUE, SON PROCEDE DE PREPARATION ET SON UTILISATION EN COSMETIQUE**
PRODUKT, BAZIEREND AUF GEFAERBTEN MINERALTEILCHEN, DIE EIN MELANIN-PIGMENT ENTHALTEN, SEIN HERSTELLUNGSVERFAHREN UND SEINE VERWENDUNG IN DER KOSMETIK
PRODUCT BASED ON COLOURED MINERAL PARTICLES INCLUDING A MELANIC PIGMENT, PROCESS FOR PREPARING SAME AND APPLICATION IN COSMETICS

(30) Priorité: 16.01.1992 FR 9200415
(43) Date de publication de la demande: 29.12.1993
(73) Titulaire: L'OREAL, F-75008 Paris (FR)
(72) Inventeur: ANDREAN, Hervé, F-75014 Paris (FR); JUNINO, Alex, F-93190 Livry-Gargan (FR); ARRAUDEAU, Jean-Pierre, F-75015 Paris (FR)
(74) Mandataire: Casalonga, Axel
(86) Numéro de dépôt international: FR9300029
(87) Numéro de publication internationale: WO9313743

(56) Documents cités:
- EP-A- 0 220 617
- EP-A- 0 379 409
- EP-A- 0 467 767
- GB-A- 2 207 153

## Description

La présente invention est relative à un produit sous forme de particules minérales colorées comportant un ou plusieurs pigments mélaniques, à son procédé de préparation et à son utilisation, notamment dans le domaine de la cosmétique, pour le maquillage des phanères et/ou de la peau, la protection de l'épiderme humain contre les rayonnements UV.

La couleur de la peau et phanères, c'est-à-dire des cheveux, des poils et des ongles d'origine humaine provient principalement des pigments mélaniques sécrétés par les mélanocytes.

Ces pigments, d'origine naturelle, comprennent en particulier des pigments noirs ou bruns qu'on appelle les eumélanines.

Leur biosynthèse naturelle s'effectue en plusieurs étapes par polymérisation des produits d'oxydation d'un acide aminé : la tyrosine et l'un de ces produits d'oxydation est le 5,6-dihydroxyindole qui polymérise à son tour en eumélanine.

La demanderesse a décrit, dans les demandes de brevets et brevets antérieurs, différents procédés permettant de teindre les cheveux humains ou la peau avec du 5,6-dihydroxyindole ou ses dérivés, en mettant en oeuvre différents systèmes d'oxydation. Les colorants ainsi formés se fixent ou pénètrent dans le substrat kératinique.

On souhaite, à certaines occasions, pouvoir conférer aux cheveux une coloration qui puisse éventuellement être enlevée rapidement.

On utilise, par ailleurs, dans les compositions de maquillage pour la peau, les phanères tels que les poils, les cils ou les sourcils, les ongles des pigments à base de composés métalliques tels que par exemple les oxydes de fer, noir et brun.

Les pigments utilisables dans ce but sont peu nombreux et offrent une palette de coloris très limitée et on recherche de ce fait, des pigments présentant une plus grande variété de couleurs pour satisfaire aux besoins du maquillage et qui soient cosmétiquement acceptables.

Dans sa demande EP-A-O 467 767, qui n'appartient à l'état de la technique que pour déterminer la nouveauté de la présente invention, la demanderesse décrit un produit à base de particules lamellaires, organiques ou minérales, ayant une dimension inférieure à 50 µm et comportant au moins un pigment mélanique synthétique, formé in situ par oxydation d'un composé indolique. Ce produit est utilisé pour la protection de l'épiderme humain, dans les produits de maquillage et pour la coloration des cheveux.

La demanderesse vient de découvrir qu'il était possible de préparer in vitro un produit sous forme d'une poudre formée de particules minérales colorées et comportant un ou plusieurs pigments mélaniques.

Elle a découvert que l'utilisation des particules définies ci-dessus était particulièrement avantageuse dans la mesure où une fois introduites dans un milieu cosmétiquement acceptable, elles se répartissent bien dans la composition qui s'étale facilement sur les phanères ou la peau et présente un pouvoir couvrant important.

Elle a découvert également que la combinaison de pigments mélaniques avec des particules minérales colorées, conduisait à des pigments présentant une grande variété de colorations particulièrement stables à la lumière.

Elle a constaté également que ces particules colorées comportant des pigments mélaniques, présentaient un coefficient d'absorption des radiations ultraviolettes particulièrement intéressant par rapport aux produits connus jusqu'à présent.

On appelle "particules minérales colorées" des particules non blanches constituées de sels métalliques, insolubles dans le milieu cosmétique, utilisables en cosmétique, référencées dans le Color Index sous le chapitre "Inorganic Colouring Matters" et portant les numéros 77000 à 77947, à l'exclusion des pigments blancs et de particules se présentant sous forme lamellaire telle que l'oxyde de fer lamellaire. Ces particules minérales colorées peuvent être constituées d'un seul pigment ou d'un mélange de pigments et peuvent ainsi se présenter sous forme de pigments nacrés ou interférentiels.

On appellera dans la suite "pigment mélanique synthétique" des pigments formés in situ par oxydation d'un composé indolique, répondant en particulier à la formule I définie ci-après.

La présente invention a donc pour objet une poudre constituée de particules minérales colorées comportant un ou plusieurs pigments mélaniques.

Un autre objet de l'invention est constitué par la préparation d'une telle poudre.

L'invention a également pour objet l'application cosmétique de telles poudres, notamment dans les produits de maquillage de la peau et des phanères et dans la protection de l'épiderme humain contre les rayonnements UV.

D'autres objets de l'invention apparaîtront à la lecture de la description et des exemples qui suivent.

Le produit conforme à l'invention est essentiellement caractérisé par le fait qu'il se présente sous forme d'une poudre constituée de particules minérales colorées dont la dimension la plus grande est inférieure à 200 µm et comportant dans ou sur les particules, un pigment mélanique synthétique formé in situ.

Le pigment mélanique résulte de l'oxydation d'au moins un composé indolique, répondant à la formule : 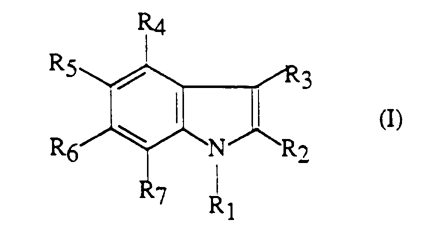 dans laquelle :
R₁ et R₃ désignent, indépendamment l'un de l'autre, un atome d'hydrogène ou un groupe alkyle en C₁-C₄;
R₂ représente un atome d'hydrogène, un groupement alkyle en C₁-C₄, un groupement carboxyle ou un groupement alcoxy(C₁-C₄) carbonyle;
R₄ et R₇ désignent, indépendamment l'un de l'autre, un atome d'hydrogène, un groupement hydroxy, un groupe alkyle en C₁-C₄, amino, alcoxy(C₁-C₄), acyl(C₂-C₄)oxy, acyl(C₂-C₄)amino;
R₅ désigne hydrogène, un groupement hydroxy, alcoxy(C₁-C₄), alkyle(C₁-C₄), halogène, amino, acyl(C₂-C₁₄)oxy, acyl(C₂-C₄)amino, triméthylsilyloxy;
R₆ désigne hydrogène, un groupement hydroxy, alcoxy(C₁-C₄), amino, acyl(C₂-C₄)oxy, acyl(C₂-C₄)amino, triméthylsilyloxy, hydroxyalkyl(C₂-C₄)amino;
R₅ et R₆ peuvent former, conjointement avec les atomes de carbone auxquels ils sont rattachés, un cycle méthylènedioxy éventuellement substitué par un groupement alkyle en C₁-C₄ ou alcoxy en C₁-C₄ ou un cycle carbonyldioxy; au moins l'un des groupements R₄ à R₇ représente un groupement OZ ou NHR, un seul au plus des groupements R₄ à R₇ désignant NHR;
   et au plus deux des groupements R₄ à R₇ désignent OZ, dans le cas où Z désigne hydrogène, ces groupements sont en position 5 et 6;
   et au moins un des groupements R₄ à R₇ représente hydrogène, dans le cas où un seul de ces groupements désigne hydrogène, un seul groupement parmi R₄ à R₇ désigne alors NHR ou OZ, et les autres groupements désignent alkyle en C₁-C₄;
   R désignant dans NHR un atome d'hydrogène, un groupement acyle en C₂-C₄, hydroxyalkyle en C₂-C₄ et Z désignant dans OZ un atome d'hydrogène, un groupement acyle en C₂-C₁₄, alkyle en C₁-C₄, triméthylsilyle;
   et les sels correspondants.

Les composés indoliques de formule (I) sont choisis, en particulier, parmi le 4-hydroxyindole, 5-hydroxyindole, 6-hydroxyindole, 7-hydroxyindole, le 4-hydroxy 5-méthoxyindole, le 4-hydroxy 5-éthoxyindole, le 2-carboxy 5-hydroxyindole, le 5-hydroxy 6-méthoxyindole, le 6-hydroxy 7-méthoxyindole, le 5-méthoxy 6-hydroxyindole, le 5,6-dihydroxyindole, le N-méthyl 5,6-dihydroxyindole, le 2-méthyl 5,6-dihydroxyindole, le 3-méthyl 5,6-dihydroxyindole, le 2,3-diméthyl 5,6-dihydroxyindole, le 2-carboxy 5,6-dihydroxyindole, le 4-hydroxy 5-méthylindole, le 2-carboxy 6-hydroxyindole, le 6-hydroxy N-méthylindole, le 2-éthoxycarbonyl-5,6-dihydroxyindole, le 4-hydroxy 7-méthoxy 2,3-diméthylindole, le 4-hydroxy 5-éthoxy N-méthylindole, le 6-hydroxy 5-méthoxy 2-méthylindole, le 6-hydroxy 5-méthoxy 2,3-diméthylindole, le 6-hydroxy 2-éthoxycarbonylindole, le 7-hydroxy 3-méthylindole, le 5-hydroxy 6-méthoxy 2,3-diméthylindole, le 5-hydroxy 3-méthylindole, le 5-acétoxy 6-hydroxyindole, le 5-hydroxy 2-éthoxycarbonylindole, le 6-hydroxy 2-carboxy 5-méthylindole, le 6-hydroxy 2-éthoxycarbonyl-5-méthoxyindole, le 6-N-β-hydroxyéthylaminoindole, le 4-aminoindole, 5-aminoindole, 6-aminoindole, 7-aminoindole, le N-méthyl 6-β-hydroxyéthylaminoindole, le 6-amino 2,3-diméthylindole, le 6-amino 2,3,4,5-tétraméthylindole, le 6-amino 2,3,4-triméthylindole, le 6-amino 2,3,5-triméthylindole, le 6-amino 2,3,6-triméthylindole, le 5,6-diacétoxyindole, le 5-méthoxy 6-acétoxyindole le 5,6-diméthoxyindole.

Le 5,6-dihydroxyindole, le 6-hydroxyindole, le 3-méthyl 5,6-dihydroxyindole, le 2-méthyl 5,6-dihydroxyindole, le 1-méthyl 5,6-dihydroxyindole, le 2-carboxy 5,6-dihydroxyindole, utilisés seuls ou en mélange, sont particulièrement préférés.

Les particules minérales colorées sont des particules non blanches, choisies de préférence parmi les oxydes de fer, à l'exclusion de l'oxyde de fer lamellaire, le bleu outremer (qui est un sulfosilicate complexe), les oxydes de chrome, le violet de manganèse (qui est un pyrophosphate d'ammonium et de manganèse) et le bleu de prusse (qui est un ferricyanure de fer).

La granulométrie des particules constituant la poudre conforme à l'invention est fonction de la granulométrie des particules minérales colorées de départ et peut varier dans de larges limites allant de 0,01 à 150 µm.

Lorsque la particule minérale colorée est un pigment nacré ou interférentiel, la granulométrie de la poudre comportant le pigment mélanique varie entre 10 et 150 µm.

Par contre, lorsque la particule minérale colorée est un sel métallique, tel qu'un oxyde de fer, de chrome, un sel de fer, de manganèse, la granulométrie des particules comportant le pigment mélanique constituant la poudre est généralement comprise entre 0,01 et 5 µm.

Le produit conforme à l'invention est préparé de préférence selon un procédé consistant à mélanger à l'air et à température de préférence ambiante, pouvant aller jusqu'à 100°C, le composé indolique de formule (I) et les particules minérales colorées définies ci-dessus, dans un milieu essentiellement non solvant des particules minérales colorées.

L'oxydation du composé indolique de formule (I) peut s'effectuer en milieu aqueux ou eau-solvant, à l'air en présence éventuelle d'un agent alcalin et/ou d'un catalyseur métallique d'oxydation.

Un catalyseur métallique d'oxydation préféré est constitué par l'ion cuivrique.

L'oxydation peut également s'effectuer en mettant en oeuvre le peroxyde d'hydrogène en présence d'un agent alcalin, tel que de préférence l'ammoniaque, ou en présence d'un ion iodure, l'iodure étant de préférence l'iodure d'un métal alcalin, alcalino-terreux ou d'ammonium.

On peut également procéder à l'oxydation en utilisant l'acide periodique et ses sels hydrosolubles et dérivés, les peracides organiques et leurs persels, les permanganates et bichromates, tels que de sodium ou de potassium, l'hypochlorite de sodium, les chlorites alcalins, le ferricyanure de potassium, le persulfate d'ammonium, l'oxyde d'argent, l'oxyde de plomb, le chlorure ferrique, le nitrite de sodium, les sels de terres rares, dont notamment le cérium et des oxydants organiques choisis parmi les ortho et parabenzoquinones, les ortho et parabenzoquinones monoimines ou diimines, les 1,2 et 1,4-naphtoquinones, les 1,2 et 1,4-naphtoquinones mono- ou diimines. Le sel d'acide periodique préféré est le periodate de sodium.

Il est possible d'activer les agents oxydants par un agent modificateur de pH.

Par exemple, lors de l'utilisation du système iodure/peroxyde d'hydrogène, on met en oeuvre de préférence un milieu alcalin, ce qui permet d'activer la réaction.

Le procédé particulièrement préféré consiste à utiliser comme oxydant du peroxyde d'hydrogène à pH alcalin et dans ce cas il s'agit d'un milieu ammoniacal.

Le milieu réactionnel utilisé pour former le colorant sur et dans les particules colorées est un milieu essentiellement non solvant des particules colorées considérées. Il est de préférence constitué par de l'eau et peut éventuellement être constitué par un mélange d'eau et de solvant(s). Le solvant est choisi de telle façon qu'il solubilise rapidement le composé indolique de formule (I).

Parmi ces solvants, on peut citer à titre d'exemple, les alcools inférieurs en C₁-C₄ tels que l'alcool éthylique, l'alcool propylique ou isopropylique, l'alcool tertiobutylique, les alkylèneglycols tels que l'éthylèneglycol, le propylèneglycol, les alkyléthers d'alkylèneglycols tels que les éthers monométhylique, monoéthylique et monobutylique de l'éthylèneglycol, les monométhyléthers du propylèneglycol et du dipropylèneglycol, et le lactate de méthyle.

Lorsque le milieu est constitué par un mélange eau/solvant(s), le(s) solvant(s) est (sont) présent(s) dans des concentrations comprises entre 0,5 et 90% en poids par rapport au poids total de la composition, en particulier entre 2 et 50% en poids et de préférence entre 2 et 20% en poids.

Leur nature est choisie et leur proportion est ajustée en fonction des critères de solubilité des dérivés indoliques de formule (I) et du critère d'insolubilité des particules colorées comportant le pigment mélanique.

Dans le procédé de préparation des produits sous forme de particules conformes à l'invention, on utilise de préférence le composé indolique dans des proportions pondérales comprises entre 0,1 et 10% et de préférence entre 0,5 et 7% en poids par rapport au poids total du milieu réactionnel, la charge colorée représentant 0,05 à 70% en poids du milieu réactionnel, le reste du mélange réactionnel étant généralement constitué par de l'eau ou un mélange eau/solvant(s).

Les oxydants sont mis en oeuvre dans des quantités suffisantes pour former, par oxydation, le pigment mélanique.

Lorsqu'on utilise l'ion iodure pour former le pigment mélanique, celui-ci est utilisé de préférence dans des proportions comprises entre 0,07 et 4% et en particulier entre 0,7 et 3%, en observant un rapport composé indolique/I⁻ compris entre 0,6 et 6 et plus particulièrement compris entre 3 et 4.

Les proportions sont déterminées par rapport au poids du milieu réactionnel.

La poudre constituée de particules minérales colorées comportant le pigment mélanique est isolée du milieu réactionnel par filtration ou par centrifugation, lavée à l'eau puis, éventuellement, séchée et/ou lyophilisée.

La poudre sous forme de particules minérales colorées et comportant le pigment mélanique tel que défini ci-dessus, peut être additionnée dans des supports cosmétiques classiques à une concentration comprise entre 0,05 et 35% en poids et de préférence entre 0,1 et 20% en poids par rapport au poids total de la composition pour conduire à des compositions cosmétiques protectrices de l'épiderme humain, des produits de maquillage tels que des cils, des sourcils, de la peau, des cheveux ou des ongles, comme des fards à paupières, fards à joues, ligneurs encore appelés "eye-liners", des mascaras pour les cils et les sourcils, des vernis à ongles, ou encore des compositions de coloration temporaire des cheveux. Ces supports cosmétiques sont connus en eux-mêmes.

Le milieu utilisé dans ces différentes compositions cosmétiques est un milieu essentiellement non solvant des particules minérales colorées, comportant le pigment mélanique.

On appelle milieu essentiellement non solvant, un milieu qui dissout moins de 1% en poids des particules minérales colorées.

Les compositions peuvent se présenter en particulier sous forme de lotion, de lotion épaissie, de gel, de crème, de lait, de poudre, de stick et éventuellement être conditionnées en aérosol et se présenter sous forme de mousse ou de spray.

Lorsque les compositions sont utilisées pour le maquillage de la peau, des cheveux, des cils et des sourcils, elles peuvent notamment se présenter sous forme solide ou pâteuse, anhydre ou aqueuse, comme des émulsions huile-dans-eau ou eau-dans-huile ou encore des suspensions. Ces compositions présentent l'avantage d'être stables et de présenter une bonne innocuité.

Lorsque les compositions sont utilisées pour la protection de l'épiderme humain contre les rayonnements UV, elles constituent des compositions dites "solaires" et elles peuvent se présenter sous forme de suspensions ou de dispersions dans des solvants ou des corps gras, ou encore sous forme d'émulsions telles que crèmes et laits, de pommades, de gels, de bâtonnets solides ou de mousses aérosols.

Dans tous les cas, lorsqu'elles sont utilisées sous forme d'émulsions, elles peuvent contenir en outre des agents tensio-actifs bien connus dans l'état de la technique, tels que des agents tensio-actifs anioniques, non-ioniques, cationiques ou amphotères.

Les compositions de maquillage et les compositions solaires peuvent également contenir des corps gras, des solvants organiques, des silicones, des épaississants, des adoucissants, des filtres solaires, des agents anti-mousses, des agents hydratants, des parfums, des conservateurs, des agents anti-oxydants, des charges, des séquestrants, des agents de traitement tels que des polymères anioniques, cationiques, non ioniques, amphotères, ou leurs mélanges, des propulseurs, des agents alcalinisants ou acidifiants.

Les corps gras peuvent être constitués par une huile ou une cire ou leur mélange, les acides gras, les alcools gras, la vaseline, la paraffine, la lanoline, la lanoline hydrogénée, la lanoline acétylée.

Les huiles sont choisies parmi les huiles animales, végétales, minérales ou de synthèse et notamment l'huile de palme hydrogénée, l'huile de ricin hydrogénée, l'huile de vaseline, l'huile de paraffine, l'huile de Purcellin.

Les cires sont choisies parmi les cires animales, fossiles, végétales, minérales ou de synthèse. On peut citer notamment les cires d'abeilles, les cires de Carnauba, de Candellila, de canne à sucre, du Japon, les ozokérites, la cire de Montan, les cires microcristallines, les paraffines.

Lorsque les compositions sont utilisées pour la coloration des ongles, elles se présentent sous forme de produits dits "vernis à ongles" contenant la poudre conforme à l'invention sous forme dispersée dans un solvant cosmétiquement acceptable contenant une ou plusieurs résines et des ingrédients habituellement utilisés dans ce type de produit.

Les compositions conformes à l'invention peuvent également contenir en plus des particules minérales colorées comportant des pigments mélaniques, telles que définies ci-dessus, d'autres pigments généralement utilisés en cosmétique, notamment des pigments blancs, des pigments nacrés et/ou nacrants permettant de varier les colorations susceptibles d'être obtenues, ou d'augmenter la protection vis-à-vis du rayonnement ultraviolets. Dans ce dernier cas, on utilise des nanopigments d'oxydes métalliques tels que les oxydes de titane, de zinc, de cérium ou de zirconium, de diamètre moyen inférieur à 100 nm et de préférence compris entre 5 et 50 nm. Les nanopigments peuvent être enrobés ou non enrobés.

Les pigments enrobés sont des pigments qui ont subi un ou plusieurs traitements de surface de nature chimique, électronique, mécanochimique, et/ou mécanique avec des composés tels que décrits par exemple dans COSMETICS & TOILETRIES, Février 1990, Vol. 105, pages 53-64, tels que des aminoacides, de la cire d'abeille, des acides gras, des alcools gras, des tensio-actifs anioniques, des lécithines, des sels de sodium, potassium, zinc, fer ou aluminium d'acides gras, des alcoxydes métalliques (de titane ou d'aluminium), du polyéthylène, des silicones, des protéines (collagène, élastine), des alcanolamines, des oxydes de silicium, des oxydes métalliques ou de l'hexamétaphosphate de sodium.

L'invention a également pour objet un procédé de coloration temporaire des cheveux, de maquillage de la peau, des cils et sourcils ou des ongles, de la protection de l'épiderme humain contre les effets néfastes des rayonnements UV, mettant en oeuvre une poudre à base de particules minérales colorées comportant des pigments mélaniques telle que définie ci-dessus, cette poudre étant appliquée directement ou au moyen de compositions cosmétiques telles que définie ci-dessus.

Les exemples suivants sont destinés à illustrer l'invention sans pour autant présenter un caractère limitatif.

A la connaissance de la Demanderesse, les dénominations des produits utilisés dans les exemples écrites en majuscules sont des marques déposées.

### EXEMPLES DE PREPARATION

### EXEMPLE 1

On solubilise 20 g (0,134 mole) de 5,6-dihydroxyindole dans 400 ml de solution aqueuse à 0,1% d'ammoniaque. On ajoute à ce mélange 180 g de bleu outremer (CI 77007). On agite 15 minutes la suspension, puis on la porte à 80°C. On additionne en 60 minutes 134,57 g d'eau oxygénée contenant 10,2 g (0,3 mole) de peroxyde d'hydrogène en maintenant la température entre 80 et 85°C. L'addition terminée, on maintient la température à 80°C pendant 2 heures, puis on refroidit le milieu réactionnel à 10°C. On essore le produit et on le lave à l'eau. Après séchage, on obtient 199 g de poudre bleu foncé.

### EXEMPLE 2

On solubilise 20 g (0,134 mole) de 5,6-dihydroxyindole dans 100 mi de solution aqueuse à 0,1% d'ammoniaque. On ajoute à ce mélange 180 g d'oxyde de chrome hydraté (CI 77289). On agite 15 minutes la suspension, puis on la porte à 80°C. On additionne en 60 minutes 134,57 g d'eau oxygénée contenant 10,2 g (0,3 mole) de peroxyde d'hydrogène en maintenant la température entre 80 et 85°C. L'addition terminée, on maintient la température à 80°C pendant 2 heures, puis on refroidit le milieu réactionnel à 10°C. On essore le produit et on le lave à l'eau. Après séchage, on obtient 198 g de poudre vert foncé.

### EXEMPLE 3

On solubilise 23,34 g (0,156 mole) de 5,6-dihydroxyindole dans 486 ml de solution aqueuse à 0,1% d'ammoniaque. On ajoute à ce mélange 210 g d'oxyde de fer rouge qui est un mélange d'oxyde de fer jaune (CI 77492) et d'oxyde de fer brun (CI 77491) et on agite 15 minutes la suspension, puis on la porte à 80°C. On additionne en 1 heure 168 g d'eau oxygénée contenant 12,59 g (0,37 mole) de peroxyde d'hydrogène en maintenant la température entre 80 et 85°C. L'addition terminée, on maintient la température à 80°C pendant 1 heure, puis on refroidit le milieu réactionnel à 20°C. On essore le précipité et on le lave à l'eau. Après séchage, on obtient 229 g de poudre brun foncé.

### EXEMPLE 4

On solubilise 20 g (0,134 mole) de 5,6-dihydroxyindole dans 400 ml de solution aqueuse à 0,1% d'ammoniaque. On ajoute à ce mélange 180 g d'oxyde de chrome (CI 77288). On agite 15 minutes la suspension puis on la porte à 80°C. On additionne en une heure 134,6 g d'eau oxygénée contenant 10,2 g (0,3 mole) de peroxyde d'hydrogène en maintenant la température entre 80 et 85°C. L'addition terminée, on maintient la temperature à 80°C pendant 2 heures puis on refroidit le milieu réactionnel à 20°C. On centrifuge le produit et on le lave à l'eau. Après lyophilisation, on obtient 195 g de poudre vert foncé.

### EXEMPLE 5

On solubilise 20 g (0,123 mole) de 5,6-dihydroxy 3-méthylindole dans 400 ml de solution aqueuse à 0,1% d'ammoniaque. On ajoute à ce mélange 180 g d'oxyde de fer noir (CI 77499). On agite 15 minutes la suspension puis on la porte à 80°C. On additionne en une heure 134 g d'eau oxygénée contenant 9,34 g de peroxyde d'hydrogène en maintenant la température entre 80° et 85°C. L'addition terminée, on maintient la température à 80°C pendant 2 heures puis on refroidit le mélange réactionnel à 20°C. On centrifuge le produit et le lave à l'eau. Après lyophilisation, on obtient 196 g de poudre brun foncé.

### EXEMPLE 6

On solubilise 3 g (2.10⁻² mole) de 5,6-dihydroxyindole dans 200 ml de solution aqueuse à 0,1% d'ammoniaque. On ajoute à ce mélange 97 g de bleu de prusse (CI 77510). On agite 15 minutes la suspension puis on la porte à 80°C. On additionne en 30 minutes 20,2 g d'eau oxygénée contenant 1,55 g (4,57.10⁻² mole) de peroxyde d'hydrogène en maintenant la température entre 80° et 85°C. L'addition terminée, on maintient la température à 80°C pendant une heure puis on refroidit le milieu réactionnel à 20°C. On centrifuge le produit et on le lave à l'eau. Après lyophilisation, on obtient 88,5 g de poudre bleue.

### EXEMPLE 7

On solubilise 10 g (0,067 mole) de 5,6-dihydroxyindole dans 400 ml de solution aqueuse à 0,1% d'ammoniaque. On ajoute à ce mélange 190 g de mica-titane vendu par la Société MERCK sous le nom de Colorona dark blue (mélange des pigments : CI 77019, CI 77891, CI 77510). On agite 15 minutes la suspension puis on la porte à 80°C. On additionne en 30 minutes 67,3 g d'eau oxygénée contenant 5,19 g (0,15 mole) de peroxyde d'hydrogène en maintenant la température entre 80° et 85°C. L'addition terminée, on maintient la température à 80°C pendant 2 heures puis on refroidit le milieu réactionnel. On centrifuge le produit et on le lave à l'eau. Après lyophilisation, on obtient 199 g de poudre bleu foncé.

### EXEMPLE 8

On solubilise 4,4 g (0,03 mole) de 5,6-dihydroxyindole dans 88 ml de solution aqueuse d'ammoniaque à 0,1%. On ajoute à ce mélange 40 g d'oxyde de fer noir. On agite la suspension pendant 15 minutes puis on la porte à 80°C. On additionne en 60 minutes 26,8 g d'eau oxygénée contenant 6,7 g (0,2 mole) de peroxyde d'hydrogène en maintenant la température entre 80°C et 85°C. L'addition terminée, on maintient la température à 80°C pendant environ 2 heures puis on refroidit le milieu réactionnel avec de la glace. La suspension obtenue est centrifugée puis lavée à l'eau. Après lyophilisation, on obtient 41,07 g de poudre noire.

### EXEMPLE 9

On solubilise 5 g (0,034 mole) de 5,6-dihydroxyindole dans 200 ml de solution aqueuse à 0,1 % d'ammoniaque. On ajoute à ce mélange 95 g de bleu outremer. On agite la suspension pendant 15 minutes puis on la porte à 80°C. On additionne 30,4 g d'eau oxygénée contenant 2,3 g (0,067 mole) de peroxyde d'hydrogène en maintenant la température entre 80 et 85°C. L'addition terminée, on maintient la température à 80°C et l'agitation pendant 2 heures puis on refroidit le milieu réactionnel à 20°C. On centrifuge le produit et on le lave à l'eau. Après lyophilisation, on obtient 91,11 g de poudre bleue.

### EXEMPLE 10

On solubilise 2,5 g (0,017 mole) de 5,6-dihydroxyindole dans 200 ml de solution aqueuse d'ammoniaque à 0,1%. On ajoute à ce mélange 97,5 g de bleu outremer. On agite la suspension pendant 15 minutes puis on la porte à 80°C. On additionne par portion 15,2 g d'eau oxygénée contenant 1,22 g (0,036 mole) de peroxyde d'hydrogène en maintenant la température entre 80 et 85°C. L'addition terminée, on maintient l'agitation et la température à 80°C pendant 2 heures 1/2. On refroidit le milieu réactionnel à 20°C. On centrifuge le produit et on le lave à l'eau. Après lyophilisation, on obtient 91,85 g de poudre bleue.

### EXEMPLE 11

On solubilise 1 g (6,7.10⁻³ mole) de 5,6-dihydroxyindole dans 200 ml de solution aqueuse d'ammoniaque à 0,1%. On ajoute à ce mélange 99 g de bleu outremer. On agite la suspension pendant 15 minutes puis on la porte à 80°C. On additionne par portion 6,1 g d'eau oxygénée contenant 0,44 g (0,013 mole) de peroxyde d'hydrogène en maintenant la température entre 80 et 85°C. L'addition terminée, on maintient l'agitation et la température à 80°C pendant 2 heures 1/2. On refroidit le milieu réactionnel à 20°C. On centrifuge le produit et on le lave à l'eau. Après lyophilisation, on obtient 96,18 g de poudre bleue.

### EXEMPLE 12

On solubilise 5 g (0,034 mole) de 5,6-dihydroxyindole dans 200 ml de solution aqueuse d'ammoniaque à 0,1%. On ajoute à ce mélange 95 g d'oxyde de chrome hydraté. On agite la suspension pendant 15 minutes puis on la porte à 80°C. On procède ensuite comme à l'exemple 9. Après lyophilisation, on obtient 96,87 g de poudre vert foncé.

### EXEMPLE 13

On solubilise 5 g (0,034 mole) de 5,6-dihydroxyindole dans 200 ml de solution aqueuse d'ammoniaque à 0,1 %. On ajoute à ce mélange 95 g d'oxyde de fer rouge qui est un mélange d'oxyde de fer jaune (CI 77492) et d'oxyde de fer brun (CI 77491) et on agite la suspension pendant 15 minutes puis on la porte à 80°C. On procède ensuite comme à l'exemple 9. Après lyophilisation, on obtient 98,09 g de poudre brun.

### EXEMPLE 14

On solubilise 2,5 g (0,017 mole) de 5,6-dihydoxyindole dans 200 ml de solution aqueuse d'ammoniaque à 0,1%. On ajoute à ce mélange 97,5 g d'oxyde de fer rouge tel que décrit à l'exemple précédant et on agite la suspension pendant 15 minutes puis on la porte à 80°C. On procède ensuite comme à l'exemple 10. Après lyophilisation, on obtient 83,2 g de poudre brun.

### EXEMPLE 15

On solubilise 20 g (0,134 mole) de 5,6-dihydroxyindole dans 400 ml de solution aqueuse d'ammoniaque à 0,1%. On ajoute à ce mélange 180 g de violet de manganèse (CI 77742). On procède ensuite comme indiqué à l'exemple 1. Après séchage, on obtient 189,64 g de poudre gris violet.

### EXEMPLES DE COMPOSITIONS

### EXEMPLE DE COMPOSITION 1

### COMPOSITION POUR MAQUILLER LES CHEVEUX

On prépare la composition suivante :

| | |
|---|---|
| - Copolymère acide méthacrylique/acrylate d'éthyle réticulé en dispersion aqueuse à 38% de MA, vendu sous la dénomination VISCOATEX 538 par la Société COATEX | 3,95 g |
| - Copolymère hydroxyéthylcellulose/chlorure de diallyldiméthylammonium, vendu sous la dénomination CELQUAT LOR par la Société NATIONAL STARCH | 1,5 g |
| - Poudre colorée de l'exemple 2 | 1,0 g |
| - Conservateur | 0,15 g |
| - 2-amino 2-méthyl 1-propanol qs pH=7,5 | |
| - Eau déminéralisée | qsp 100 g |

Cette composition gélifiée appliquée sur les cheveux leur confère une teinte vert amande.

### EXEMPLE DE COMPOSITION 2

### COMPOSITION POUR MAQUILLER LES CHEVEUX

On prépare la composition suivante :

| | |
|---|---|
| - VISCOATEX 538 vendu par la Société COATEX | 3,95 g |
| - CELQUAT LOR vendu par la Société NATIONAL STARCH | 1,5 g |
| - Emulsion cationique à 35% de polydiméthylsiloxane à groupements aminoéthylpropylamine, vendue sous la dénomination DC 929 par la Société DOW CORNING | 0,85 g |
| - Poudre colorée de l'exemple 1 | 1,0 g |
| - Conservateur | 0,25 g |
| - Séquestrant | 0,2 g |
| - Ethanol | 10,0 g |
| - 2-amino 2-méthyl 1-propanol qs pH=7,5 | |
| - Eau déminéralisée | qsp 100 g |

Cette composition gélifiée est appliquée sur les cheveux et leur confère une teinte gris bleuté.

### EXEMPLE DE COMPOSITION 3

### COMPOSITION POUR LE MAQUILLAGE DES CHEVEUX

On prépare la composition suivante :

| | |
|---|---|
| - VISCOATEX 538 vendu par la Société COATEX | 3,85 g |
| - CELQUAT LOR vendu par la Société NATIONAL STARCH | 1,5 g |
| - Poudre colorée de l'exemple 3 | 1,0 g |
| - Ethanol | 10,0 g |
| - Conservateur | 0,25 g |
| - Parfum | 0,3 g |
| - 2-amino 2-méthyl 1-propanol qs pH=7,5 | |
| - Eau déminéralisée | qsp 100 g |

Cette composition gélifiée confère aux cheveux une couleur marron rouge.

### EXEMPLE DE COMPOSITION 4

On prépare un vernis à ongles de composition suivante :

| | | |
|---|---|---|
| - Nitrocellulose | | 10,90 g |
| - Résine toluène sulfonamide formaldéhyde | | 9,85 g |
| - Acétyltributyle citrate | | 6,50 g |
| - Acétate de butyle | | 21,80 g |
| - Acétate d'éthyle | | 9,40 g |
| - Alcool isopropylique | | 7,80 g |
| - Stéaralkonium hectorite | | 1,36 g |
| - Pigments : | . Ultramarine blue | 0,01 g |
| | . Dioxyde de titane | 0,27 g |
| | . DC Red 34 | 0,07 g |
| | . Poudre colorée de l'exemple 3 | 0,30 g |
| | . Agents nacrants (mica-titane) | 0,60 g |
| - Acide citrique | | 0,06 g |
| - Toluène | | qsp 100 g |

Ce vernis confère aux ongles une couleur brun nacré.

### EXEMPLE DE COMPOSITION 5

On prépare un fond de teint de composition suivante :

| | |
|---|---|
| - Stéarate de glycéryle | 2,2 g |
| - Mélange d'acides caprique et caprylique et de triester de glycérine | 15,0 g |
| - Poudre colorée de l'exemple 3 | 9,0 g |
| - Parahydroxybenzoate de méthyle | 0,1 g |
| - Parahydroxybenzoate de propyle | 0,1 g |
| - Conservateur | 0,3 g |
| - 2-hydroxy 4-méthoxybenzophénone | 0,5 g |
| - Octyldiméthyl p-aminobenzoate | 0,5 g |
| - Silicate de magnésium et d'aluminium | 1,0 g |
| - Triéthanolamine | 1,0 g |
| - Carboxyméthylcellulose | 0,16 g |
| - Sel d'aluminium du produit de réaction de l'anhydride octénylsuccinique et de l'amidon, vendu sous la dénomination DRY FLO par la Société NATIONAL STARCH | 5,0 g |
| - Polydiméthylsiloxane cyclique vendue sous la dénomination SILBIONE OIL 70045 par la Société RHONE POULENC | 10,0 g |
| - Propylèneglycol | 2,0 g |
| - Glycérine | 3,0 g |
| - Lauroylsarcosinate de sodium | 0,6 g |
| - Acide stéarique | 2,2 g |
| - Eau | qsp 100 g |

Le fond de teint obtenu est beige naturel.

### EXEMPLE DE COMPOSITION 6

On prépare une poudre corporelle compactée de composition suivante :

| | |
|---|---|
| - Stéarate de zinc | 6,0 g |
| - Parahydroxybenzoate de propyle | 0,2 g |
| - Parfum | 4,0 g |
| - Poudre colorée de l'exemple 3 | 0,07 g |
| - Talc | qsp 100 g |

Cette poudre beige naturel s'applique sur le corps avec une houppe.

### EXEMPLE DE COMPOSITION 7

On prépare un fard à paupières sous forme de poudre compactée, de composition suivante :

| | |
|---|---|
| - Poudre de polyamide | 15,0 g |
| - Polydiméthylsiloxane cyclique vendu sous la dénomination SILBIONE OIL par la Société RHONE POULENC | 9,0 g |
| - Agent nacrant : Mica-titane | 30,0 g |
| - Poudre colorée de l'exemple 2 | 7,0 g |
| - Poudre colorée de l'exemple 1 | 6,0 g |
| - Talc | qsp 100 g |

Ce fard à paupières a une couleur bleu-vert et s'applique à l'aide d'un pinceau ou d'un applicateur en mousse.

### EXEMPLE DE COMPOSITION 8

On prépare un mascara waterproof de composition suivante :

| | |
|---|---|
| - Cire de Carnauba | 5,0 g |
| - Cire de Candelilla | 5,0 g |
| - Alcool éthylique | 3,0 g |
| - Montmorillonite modifiée avec une substance organique | 4,0 g |
| - Lanoline | 2,0 g |
| - Talc | 10,0 g |
| - Poudre colorée de l'exemple 3 | 10,0 g |
| - Isoparaffine | qsp 100 g |

Le mode opératoire est le suivant :

On chauffe les cires à 80°C. On ajoute le talc et les pigments. On incorpore ensuite la Montmorillonite qui a été modifiée avec une substance organique et une partie de l'isoparaffine. A environ 40°C, on introduit l'alcool éthylique et le reste de l'isoparaffine. On passe le tout à la broyeuse.

Ce masquara waterproof est facile à appliquer et colore les cils en brun.

### EXEMPLE DE COMPOSITION 9

On prépare un mascara de composition suivante :

| | |
|---|---|
| - Stéarate de triéthanolamine | 15,0 g |
| - Cire d'abeilles | 8,0 g |
| - Paraffine | 3,0 g |
| - Colophane | 2,0 g |
| - Ozokérite | 10,0 g |
| - Parahydroxybenzoate de propyle | 0,20 g |
| - Parahydroxybenzoate de méthyle | 0,20 g |
| - Gomme arabique | 0,50 g |
| - Hydrolysat de kératine | 1,0 g |
| - Oxyde de fer noir | 5,0 g |
| - Poudre colorée de l'exemple 2 | 5,0 g |
| - Eau | qsp 100 g |

Ce mascara sous forme d'émulsion est préparé de la façon suivante :

On fait fondre les cires. On incorpore les pigments. On fait chauffer la phase aqueuse contenant la gomme arabique et l'hydrolysat de kératine à la même température que la phase cireuse. On mélange les deux phases et o n agite vigoureusement.

Le mascara obtenu confère aux cils une couleur bleu gris avec de légers reflets métalliques.

### EXEMPLE DE COMPOSITION 10

On prépare un fard à joues sous forme de poudre compactée, de composition suivante :

| | |
|---|---|
| - Dioxyde de titane | 10,0 g |
| - Mica-titane | 10,0 g |
| - DC Red 30 | 1,2 g |
| - Parahydroxybenzoate de propyle | 0,2 g |
| - Huile de vaseline | 6,0 g |
| - 2-hydroxy 4-méthoxybenzophénone vendue sous la dénomination UVINUL M-40 par la Société BASF | 0,5 g |
| - Poudre colorée de l'exemple 3 | 3,0 g |
| - Talc | qsp 100 g |

Ce fard à joues de couleur brun orangé s'applique avec un pinceau.

### EXEMPLE DE COMPOSITION 11

On prépare une poudre compacte pour le visage de composition suivante :

| | |
|---|---|
| - Poudre de polyéthylène | 5,0 g |
| - Poudre colorée de l'exemple 3 | 6,0 g |
| - Dioxyde de titane | 10,0 g |
| - Mica | 15,0 g |
| - Myristate d'isopropyle | 1,5 g |
| - Huile de vaseline | 1,5 g |
| - Sorbitol | 0,5 g |
| - Talc | qsp 100 g |

Cette poudre de couleur beige naturel s'applique sur le visage à l'aide d'une houpette ou d'un pinceau.

### EXEMPLE DE COMPOSITION 12

On prépare une composition anti-solaire contenant les constituants ci-après :

| | |
|---|---|
| - Mélange d'alcool cétylstéarylique et d'alcool cétylstéarylique oxyéthyléné à 33 moles d'oxyde d'éthylène (80/20) | 7,0 g |
| - Stéarate de glycéryle vendu sous la dénomination GELEOL par la Société GATTEFOSSE | 2,0 g |
| - Alcool cétylique pur | 1,5 g |
| - Polydiméthylsiloxane vendu sous la dénomination SILBIONE OIL 70047 V 300 par la Société RHONE POULENC | 1,5 g |
| - Huile de vaseline | 15,0 g |
| - p-hydroxybenzoate de butyle | 0,2 g |
| - Poudre colorée de l'exemple 3 | 0,15 g |
| - Glycérol | 20,0 g |
| - Imidazolidinylurée | 0,2 g |
| - Eau déminéralisée stérilisée | qsp 100 g |

### EXEMPLE DE COMPOSITION 13

On prépare une composition de maquillage des cils contenant les constituants ci-après :

| | |
|---|---|
| - Copolymère d'acrylates/acrylate d'alkyle en C₈-C₃₀ réticulé | 0,1 g |
| - Copolymère carboxyvinylique réticulé, vendu sous la dénomination CARBOPOL 940 par la Société GOODRICH CHEMICAL | 0,6 g |
| - Triéthanolamine | 0,8 g |
| - Glycérine | 2,0 g |
| - Conservateur | 0,2 g |
| - Octaméthyl cyclosiloxane | 25,0 g |
| - Oxyde de fer noir | 5,0 g |
| - Poudre colorée de l'exemple 9 | 5,0 g |
| - Eau | qsp 100 g |

Les polymères sont dispersés à chaud avec les conservateurs dans l'eau pour faire un gel. La glycérine et la triéthanolamine sont ajoutées. Le pigment est dispersé dans la silicone et ajouté dans la phase gélifiée.

On obtient une émulsion gélifiée bleu marine pour le maquillage des cils.

### EXEMPLE DE COMPOSITION 14

On prépare un rouge à lèvres de composition suivante :

| | |
|---|---|
| - 2,6-di-tert-butyl-p-crésol | 0,16 g |
| - Lanoline liquide | 17,5 g |
| - Cire microcristalline | 15,0 g |
| - Triglycérides d'acides carprylique et caprique | 11,0 g |
| - Béhénate d'octyl glycéryl | 11,0 g |
| - Poudre colorée de l'exemple 14 | 3,0 g |
| - Mica-titane | 6,0 g |
| - Huile de ricin | qsp 100 g |

On obtient un rouge à lèvres de couleur brun nacré.

### EXEMPLE DE COMPOSITION 15

On prépare un fard à joues sous forme de poudre compactée, de composition suivante :

| | |
|---|---|
| - Dioxyde de titane | 10,0 g |
| - Mica-titane | 10,0 g |
| - DC Red 30 | 1,2 g |
| - Parahydroxybenzoate de propyle | 0,2 g |
| - Huile de vaseline | 6,0 g |
| - 2-hydroxy 4-méthoxybenzophénone, vendue sous la dénomination UVINUL M-40 par la Société BASF | 0,5 g |
| - Poudre colorée de l'exemple 13 | 1,0 g |
| - Talc | qsp 100 g |

Ce fard à joues de couleur brun orangé s'applique avec un pinceau.

### EXEMPLE DE COMPOSITION 16

On prépare un fond de teint de composition suivante :

| | |
|---|---|
| - Stéarate de glycéryle | 2,2 g |
| - Mélange d'acides caprique et caprylique et de triester de glycérine | 15,0 g |
| - Oxyde de titane | 10,53 g |
| - Oxyde de fer jaune | 0,83 g |
| - Poudre colorée de l'exemple 5 | 0,14 g |
| - Poudre colorée de l'exemple 14 | 0,50 g |
| - Parahydroxybenzoate de méthyle | 0,1 g |
| - Parahydroxybenzoate de propyle | 0,1 g |
| - Conservateur | 0,3 g |
| - 2-hydroxy 4-méthoxybenzophénone | 0,5 g |
| - Octyldiméthyl p-aminobenzoate | 0,5 g |
| - Silicate de magnésium et d'aluminium | 1,0 g |
| - Triéthanolamine | 1,0 g |
| - Carboxyméthylcellulose | 0,16 g |
| - Sel d'aluminium du produit de réaction de l'anhydride octénylsuccinique et de l'amidon, vendu sous la dénomination DRY FLO par la Société NATIONAL STARCH | 5,0 g |
| - Polydiméthylsiloxane cyclique, vendue sous la dénomination SILBIONE OIL 70045 par la Société RHONE POULENC | 10,0 g |
| - Propylèneglycol | 2,0 g |
| - Glycérine | 3,0 g |
| - Lauroylsarcosinate de sodium | 0,6 g |
| - Acide stéarique | 2,2 g |
| - Eau | qsp 100 g |

Le fond de teint obtenu est beige naturel.

### EXEMPLE DE COMPOSITION 17

On prépare un fard à paupières sous forme de poudre compactée, de composition suivante :

| | |
|---|---|
| - Poudre de polyamide | 15,0 g |
| - Polydiméthylsiloxane cyclique, vendu sous la dénomination SILBIONE OIL par la Société RHONE POULENC | 9,0 g |
| - Agent nacrant : Mica-titane | 30,0 g |
| - Poudre colorée de l'exemple 4 | 7,0 g |
| - Poudre colorée de l'exemple 15 | 6,0 g |
| - Talc | qsp 100 g |

Ce fard à paupières a une couleur vert-jaune et s'applique à l'aide d'un pinceau ou d'un applicateur en mousse.

## Revendications

1. Produit sous forme de poudre constitué de particules, caractérisé par le fait que les particules sont des particules non blanches et colorées, ayant une dimension inférieure à 200 µm, à l'exclusion de l'oxyde de fer lamellaire et des particules lamellaires de dimension inférieure à 50 µm, et comportant dans et/ou sur les particules un pigment mélanique synthétique, formé in situ par oxydation d'un composé indolique.

2. Produit selon la revendication 1, caractérisé par le fait que le composé indolique répond à la formule (I) : 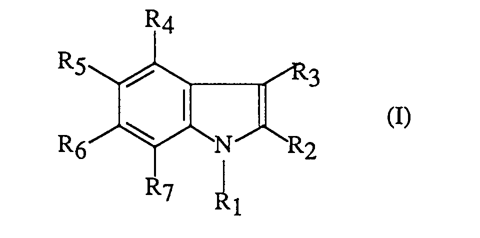 dans laquelle :
R₁ et R₃ désignent, indépendamment l'un de l'autre, un atome d'hydrogène ou un groupe alkyle en C₁-C₄;
R₂ représente un atome d'hydrogène, un groupement alkyle en C₁-C₄, un groupement carboxyle ou un groupement alcoxy(C₁-C₄) carbonyle;
R₄ et R₇ désignent, indépendamment l'un de l'autre, un atome d'hydrogène, un groupement hydroxy, un groupement alkyle en C₁-C₄, amino, alcoxy(C₁-C₄), acyl(C₂-C₄)oxy, acyl(C₂-C₄)amino;
R₅ désigne un atome d'hydrogène, un groupement hydroxy, alcoxy (C₁-C₄), alkyle(C₁-C₄), halogène, amino, acyl(C₂-C₁₄)oxy, acyl (C₂-C₄)amino, triméthylsilyloxy;
R₆ désigne hydrogène, un groupement hydroxy, alcoxy(C₁-C₄), amino, acyl(C₂-C₄)oxy, acyl(C₂-C₄)amino, triméthylsilyloxy, hydroxyalkyl(C₂-C₄)amino;
R₅ et R₆ peuvent former, conjointement avec les atomes de carbone auxquels ils sont rattachés, un cycle méthylènedioxy éventuellement substitué par un groupement alkyle en C₁-C₄ ou alcoxy en C₁-C₄ ou un cycle carbonyldioxy; au moins l'un des groupements R₄ à R₇ représente un groupement OZ ou NHR, un seul au plus des groupements R₄ à R₇ désignant NHR;
et au plus deux des groupements R₄ à R₇ désignent OZ, dans le cas où Z désigne hydrogène, ces groupements sont en position 5 et 6;
et au moins un des groupements R₄ à R₇ représente hydrogène, dans le cas où un seul de ces groupements désigne hydrogène, un seul groupement parmi R₄ à R₇ désigne alors NHR ou OZ, et les autres groupements désignent alkyle en C₁-C₄;
R désignant dans NHR un atome d'hydrogène, un groupement acyle en C₂-C₄, hydroxyalkyle en C₂-C₄ et Z désignant dans OZ un atome d'hydrogène, un groupement acyle en C₂-C₁₄, alkyle en C₁-C₄, triméthylsilyle;
et les sels correspondants.

3. Produit selon les revendidations 1 ou 2, caractérisé par le fait que le composé indolique est choisi parmi les composés suivants : le 4-hydroxyindole, 5-hydroxyindole, 6-hydroxyindole, 7-hydroxyindole, le 4-hydroxy 5-méthoxyindole, le 4-hydroxy 5-éthoxyindole, le 2-carboxy 5-hydroxyindole, le 5-hydroxy 6-méthoxyindole, le 6-hydroxy 7-méthoxyindole, le 5-méthoxy 6-hydroxyindole, le 5,6-dihydroxyindole, le N-méthyl 5,6-dihydroxyindole, le 2-méthyl 5,6-dihydroxyindole, le 3-méthyl 5,6-dihydroxyindole, le 2,3-diméthyl 5,6-dihydroxyindole, le 2-carboxy 5,6-dihydroxyindole, le 4-hydroxy 5-méthylindole, le 2-carboxy 6-hydroxyindole, le 6-hydroxy N-méthylindole, le 2-éthoxycarbonyl 5,6-dihydroxyindole, le 4-hydroxy 7-méthoxy 2,3-diméthylindole, le 4-hydroxy 5-éthoxy N-méthylindole, le 6-hydroxy 5-méthoxy 2-méthylindole, le 6-hydroxy 5-méthoxy 2,3-diméthylindole, le 6-hydroxy 2-éthoxycarbonylindole, le 7-hydroxy 3-méthylindole, le 5-hydroxy 6-méthoxy 2,3-diméthylindole, le 5-hydroxy 3-méthylindole, le 5-acétoxy 6-hydroxyindole, le 5-hydroxy 2-éthoxycarbonylindole, le 6-hydroxy 2-carboxy 5-méthylindole, le 6-hydroxy 2-éthoxycarbonyl 5-méthoxyindole, le 6-N-β-hydroxyéthylaminoindole, le 4-aminoindole, 5-aminoindole, 6-aminoindole, 7-aminoindole, le N-méthyl 6-β-hydroxyéthylaminoindole, le 6-amino 2,3-diméthylindole, le 6-amino 2,3,4,5-tétraméthylindole, le 6-amino 2,3,4-triméthylindole, le 6-amino 2,3,5-triméthylindole, le 6-amino 2,3,6-triméthylindole, le 5,6-diacétoxyindole, le 5-méthoxy 6-acétoxyindole, le 5,6-diméthoxyindole.

4. Produit selon l'une quelconque des revendications 1 à 3, caractérisé par le fait que le composé indolique est le 5,6-dihydroxyindole, le 6-hydroxyindole, le 3-méthyl 5,6-dihydroxyindole, le 2-méthyl 5,6-dihydroxyindole, le 1-méthyl 5,6-dihydroxyindole, le 2-carboxy 5,6-dihydroxyindole ou leur mélange.

5. Produit selon l'une quelconque des revendications 1 à 4, caractérisé par le fait que les particules minérales colorées sont choisies parmi les oxydes de fer, à l'exclusion de l'oxyde de fer lamellaire, le sulfosilicate complexe, les oxydes de chrome, les pyrophosphates d'ammonium et de manganèse, le ferricyanure de fer.

6. Produit selon l'une quelconque des revendications 1 à 5, caractérisé par le fait que les particules minérales colorées ont une dimension comprise entre 0,01 et 150 µm.

7. Procédé de préparation d'un produit tel que défini dans l'une quelconque des revendications 1 à 6, caractérisé par le fait que l'on mélange dans un milieu aqueux un composé indolique tel que défini dans les revendications 2, 3 ou 4 et des particules minérales colorées, ayant une dimension inférieure à 200 µm et qu'on procède ensuite à la formation du pigment mélanique par oxydation du composé indolique de formule (I).

8. Procedé selon la revendication 7, caractérisé par le fait que l'oxydation s'effectue lentement à l'air à pH alcalin.

9. Procédé selon la revendication 7, caractérisé par le fait que l'oxydation s'effectue par l'oxygène de l'air en présence d'un catalyseur métallique.

10. Procédé selon la revendication 7, caractérisé par le fait que l'oxydation s'effectue par l'addition d'agents oxydants constitués par le peroxyde d'hydrogène; les peracides organiques et leurs persels, l'acide périodique et ses sels, les permanganates, les bichromates, l'hypochlorite de sodium, les chlorites alcalins, le ferricyanure de potassium, le persulfate d'ammonium, l'oxyde d'argent, l'oxyde de plomb, le chlorure ferrique, le nitrite de sodium, les sels de terres rares et les oxydants organiques choisis parmi les ortho et parabenzoquinones, les ortho et parabenzoquinones monoimines ou diimines, les 1,2 et 1,4-naphtoquinones, les 1,2 et 1,4-naphtoquinones mono- ou diimines.

11. Procédé selon la revendication 7, caractérisé par le fait que l'oxydation s'effectue par le peroxyde d'hydrogène en milieu ammoniacal.

12. Procédé selon l'une quelconque des revendications 7 à 11, caractérisé par le fait que le milieu réactionnel est un milieu essentiellement non solvant des particules et solvant du composé indolique de formule (I), et qu'il est constitué par de l'eau ou un mélange d'eau et de solvant(s).

13. Procédé selon l'une quelconque des revendications 7 à 12, caractérisé par le fait que le solvant est choisi parmi l'alcool éthylique, l'alcool propylique ou isopropylique, l'alcool tertiobutylique, les alkylèneglycols, les alkyléthers d'alkylèneglycols et le lactate de méthyle.

14. Procédé selon l'une quelconque des revendications 7 à 13, caractérisé par le fait que le composé indolique est utilisé dans des proportions en poids comprises entre 0,1 et 10% et de préférence entre 0,5 et 7% en poids, par rapport au poids total du milieu réactionnel et que les particules minérales colorées sont utilisées dans des proportions comprises entre 0,05 et 70% en poids.

15. Composition cosmétique, caractérisée par le fait qu'elle contient dans un milieu cosmétiquement acceptable, au moins un produit tel que défini dans l'une quelconque des revendications 1 à 6, ou préparé selon l'une quelconque des revendications 7 à 14.

16. Composition selon la revendication 15, caractérisée par le fait qu'elle se présente sous forme de lotion, de lotion épaissie, de gel, de crème, de lait, de poudre, de stick, et qu'elle est éventuellement conditionnée en aérosol sous forme de spray ou de mousse.

17. Composition selon l'une quelconque des revendications 15 et 16, caractérisée par le fait qu'elle est destinée à être utilisée pour le maquillage de la peau, des cheveux, des ongles, des cils et des sourcils, et qu'elle se présente sous forme liquide, solide ou pâteuse, anhydre ou aqueuse.

18. Composition selon l'une quelconque des revendications 15 à 17, destinée à la protection de l'épiderme humain contre les rayonnements solaires UV, caractérisée par le fait qu'elle se présente sous forme de suspension ou de dispersion dans des solvants ou des corps gras, ou sous forme d'émulsions, de pommades, de gels, de bâtonnets solides ou de mousses aérosols.

19. Composition selon l'une quelconque des revendications 15 à 18, caractérisée par le fait qu'elle contient des corps gras, des solvants organiques, des silicones, des épaississants, des adoucissants, des tensio-actifs, des filtres solaires, des agents anti-mousses, des agents hydratants, des parfums, des conservateurs, des agents anti-oxydants, des charges, des séquestrants, des agents de traitement, des propulseurs, des agents alcalinisants ou acidifiants ou d'autres pigments.

20. Composition selon la revendication 19, caractérisée par le fait qu'elle contient des nanopigments d'oxydes métalliques choisis parmi les oxydes de titane, de zinc, de cérium ou de zirconium, ces nanopigments ayant un diamètre moyen inférieur à 100 nm, et sont enrobés ou non enrobés.

21. Composition selon l'une quelconque des revendications 15 à 20, caractérisée par le fait que la poudre sous forme de particules minérales colorées comportant le pigment mélanique est présente dans des concentrations comprises entre 0,05 et 35% en poids et de préférence entre 0,1 et 20% en poids par rapport au poids total de la composition.

22. Utilisation du produit tel que défini dans l'une quelconque des revendications 1 à 6 ou préparé selon le procédé défini dans l'une quelconque des revendications 7 à 14, dans la protection de l'épiderme humain.

23. Utilisation du produit tel que défini dans l'une quelconque des revendications 1 à 6 ou préparé selon le procédé défini dans l'une quelconque des revendications 7 à 14, dans des produits de maquillage de la peau, des cheveux, des ongles, des cils ou des sourcils.

## Claims

1. Product in the powder form consisting of particles, characterized in that the particles are non-white and coloured particles, having a size of less than 200 µm, with the exception of lamellar iron oxide and lamellar particles with a size less than 50 µm, and including in and/or on the particles a synthetic melanin pigment, formed in situ by oxidation of an indole compound.

2. Product according to Claim 1, characterized in that the indole compound corresponds to the formula (I): 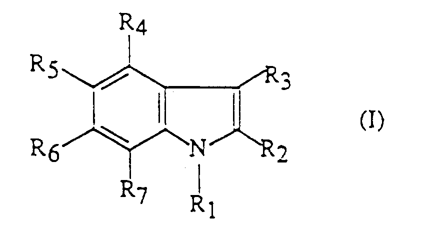 in which:
R₁ and R₃ denote, independently of each other, a hydrogen atom or a C₁-C₄ alkyl group;
R₂ represents a hydrogen atom, a C₁-C₄ alkyl group, a carboxyl group or a (C₁-C₄)alkoxycarbonyl group;
R₄ and R₇ denote, independently of each other, a hydrogen atom, a hydroxyl group, or a C₁-C₄ alkyl, amino, (C₁-C₄)alkoxy, (C₂-C₄)acyloxy or (C₂-C₄)acylamino group;
R₅ denotes hydrogen or a hydroxyl, (C₁-C₄)alkoxy, (C₁-C₄)alkyl, halo, amino, (C₂-C₁₄)acyloxy, (C₂-C₄)acylamino or trimethylsilyloxy group;
R₆ denotes hydrogen or a hydroxyl, (C₁-C₄)alkoxy, amino, (C₂-C₄)acyloxy, (C₂-C₄)acylamino, trimethylsilyloxy or hydroxy(C₂-C₄)alkylamino group;
R₅ and R₆ can form, jointly with the carbon atoms to which they are attached, a methylenedioxy ring optionally substituted by a C₁-C₄ alkyl or C₁-C₄ alkoxy group or a carbonyldioxy ring; at least one of the groups R₄ to R₇ represents an OZ or NHR group, a single one at most of the groups R₄ to R₇ denoting NHR;
and at most two of the groups R₄ to R₇ denote OZ, in the case where Z denotes hydrogen, these groups are in position 5 and 6;
and at least one of the groups R₄ to R₇ represents hydrogen, in the case where a single one of these groups denotes hydrogen, a single group from R₄ to R₇ then denotes NHR or OZ, and the other groups denote C₁-C₄ alkyl;
R denoting, in NHR, a hydrogen atom or a C₂-C₄ acyl or C₂-C₄ hydroxyalkyl group and Z denoting, in OZ, a hydrogen atom or a C₂-C₁₄ acyl, C₁-C₄ alkyl or trimethylsilyl group; and the corresponding salts.

3. Product according to Claims [sic] 1 or 2, characterized in that the indole compound is chosen from the following compounds: 4-hydroxyindole, 5-hydroxyindole, 6-hydroxyindole, 7-hydroxyindole, 4-hydroxy-5-methoxyindole, 4-hydroxy-5-ethoxyindole, 2-carboxy-5-hydroxyindole, 5-hydroxy-6-methoxyindole, 6-hydroxy-7-methoxyindole, 5-methoxy-6-hydroxyindole, 5,6-dihydroxyindole, N-methyl-5,6-dihydroxyindole, 2-methyl-5,6-dihydroxyindole, 3-methyl-5,6-dihydroxyindole, 2,3-dimethyl-5,6-dihydroxyindole, 2-carboxy-5,6-dihydroxyindole, 4-hydroxy-5-methylindole, 2-carboxy-6-hydroxyindole, 6-hydroxy-N-methylindole, 2-ethoxycarbonyl-5,6-dihydroxyindole, 4-hydroxy-7-methoxy-2,3-dimethylindole, 4-hydroxy-5-ethoxy-N-methylindole, 6-hydroxy-5-methoxy-2-methylindole, 6-hydroxy-5-methoxy-2,3-dimethylindole, 6-hydroxy-2-ethoxycarbonylindole, 7-hydroxy-3-methylindole, 5-hydroxy-6-methoxy-2,3-dimethylindole, 5-hydroxy-3-methylindole, 5-acetoxy-6-hydroxyindole, 5-hydroxy-2-ethoxycarbonylindole, 6-hydroxy-2-carboxy-5-methylindole, 6-hydroxy-2-ethoxycarbonyl-5-methoxyindole, 6-N-β-hydroxyethylaminoindole [sic] , 4-aminoindole, 5-aminoindole, 6-aminoindole, 7-aminoindole, N-methyl-6-β-hydroxyethylaminoindole [sic], 6-amino-2,3-dimethylindole, 6-amino-2,3,4,5-tetramethylindole, 6-amino-2,3,4-trimethylindole, 6-amino-2,3,5-trimethylindole, 6-amino-2,3,6-trimethylindole, 5,6-diacetoxyindole, 5-methoxy-6-acetoxyindole and 5,6-dimethoxyindole.

4. Product according to any one of Claims 1 to 3, characterized in that the indole compound is 5,6-dihydroxyindole, 6-hydroxyindole, 3-methyl-5,6-dihydroxyindole, 2-methyl-5,6-dihydroxyindole, 1-methyl-5,6-dihydroxyindole, 2-carboxy-5,6-dihydroxyindole or a mixture thereof.

5. Product according to any one of Claims 1 to 4, characterized in that the coloured inorganic particles are chosen from iron oxides, with the exception of lamellar iron oxide, complex sulfosilicate, chromium oxides, ammonium and manganese pyrophosphates and iron ferricyanide.

6. Product according to any one of Claims 1 to 5, characterized in that the coloured inorganic particles have a size between 0.01 and 150 µm.

7. Process for the preparation of product as defined in any one of Claims 1 to 6, characterized in that an indole compound as defined in Claims 2,3 or 4 and coloured inorganic particles, having a size of less than 200 µm, are mixed in an aqueous medium and in that the melanin pigment is then formed by oxidation of the indole compound of formula (I).

8. Process according to Claim 7, characterized in that oxidation is carried out slowly in air at alkaline pH.

9. Process according to Claim 7, characterized in that oxidation is carried out by the oxygen of the air in the presence of a metal catalyst.

10. Process according to Claim 7, characterized in that oxidation is carried out by the addition of oxidizing agents consisting of hydrogen peroxide, organic peracids and their persalts, periodic acid and its salts, permanganates, dichromates, sodium hypochlorite, alkaline chlorites, potassium ferricyanide, ammonium persulfate, silver oxide, lead oxide, ferric chloride, sodium nitrite, rare-earth salts and organic oxidizing agents chosen from ortho- and parabenzoquinones, ortho- and parabenzoquinone monoimines or diimines, 1,2- and 1,4-naphthoquinones, or 1,2- and 1,4-naphthoquinone mono- or diimines.

11. Process according to Claim 7, characterized in that oxidation is carried out by hydrogen peroxide in ammoniacal medium.

12. Process according to any one of Claims 7 to 11, characterized in that the reaction medium is an essentially non-solvent medium of the particles and an essentially solvent medium of the indole compound of formula (I), and in that it consists of water or a mixture of water and solvent(s).

13. Process according to any one of Claims 7 to 12, characterized in that the solvent is chosen from ethyl alcohol, propyl or isopropyl alcohol, tert-butyl alcohol, alkylene glycols, alkyl ethers of alkylene glycols and methyl lactate.

14. Process according to any one of Claims 7 to 13, characterized in that the indole compound is used in proportions by weight between 0.1 and 10 % and preferably between 0.5 and 7 % by weight with respect to the total weight of the reaction mixture and in that the coloured inorganic particles are used in proportions between 0.05 and 70 % by weight.

15. Cosmetic composition, characterized in that it contains, in a cosmetically acceptable medium, at least one product as defined in any one of Claims 1 to 6, or prepared according to any one of Claims 7 to 14.

16. Composition according to Claim 15, characterized in that it is provided in the lotion, thickened lotion, gel, cream, milk, powder or stick form and in that it is optionally packaged in an aerosol in the spray or foam form.

17. Composition according to either one of Claims 15 and 16, characterized in that it is intended to be used for making up the skin, hair, nails, eyelashes and eyebrows, and in that it is provided in the anhydrous or aqueous, liquid, solid or paste form.

18. Composition according to any one of Claims 15 to 17, intended to protect human skin against UV solar radiation, characterized in that it is provided in the foam of a suspension or dispersion in solvents or fats, or in the form of emulsions, ointments, gels, solid sticks or aerosol foams.

19. Composition according to any one of Claims 15 to 18, characterized in that it contains fats, organic solvents, silicones, thickening agents, softening agents, surface-active agents, sunscreening agents, antifoaming agents, hydrating agents, fragrances, preserving agents, antioxidizing agents, fillers, sequestering agents, treatment agents, propellants, basifying or acidifying agents or other pigments.

20. Composition according to Claim 19, characterized in that it contains nanopigments of metal oxides chosen from titanium, zinc, cerium or zirconium oxides, these nanopigments having a mean diameter of less than 100 nm, and are coated or noncoated.

21. Composition according to any one of Claims 15 to 20, characterized in that the powder in the form of coloured inorganic particles including the melanin pigment is present in concentrations between 0.05 and 35 % by weight and preferably between 0.1 and 20 % by weight with respect to the total weight of the composition.

22. Use of the product as defined in any one of Claims 1 to 6 or prepared according to the process defined in any one of Claims 7 to 14 in protecting human skin.

23. Use of the product as defined in any one of Claims 1 to 6 or prepared according to the process defined in any one of Claims 7 to 14 in products for making up the skin, hair, nails, eyelashes or eyebrows.

## Patentansprüche

1. Produkt in Form eines Pulvers aus Partikeln,
dadurch **gekennzeichnet**, daß die Partikel nicht-weiße und gefärbte Partikel mit einer Größe von weniger als 200 µm sind, ausgenommen lamellares Eisenoxid und lamellare Partikel einer Größe von weniger als 50 µm, wobei die Partikel in und/oder auf den Partikeln ein synthetisches Melanin-Pigment enthalten, das in situ durch Oxidation einer Indolverbindung gebildet ist.

2. Produkt gemäß Anspruch 1,
dadurch **gekennzeichnet**, daß die Indol-Verbindung die Formel (I) aufweist: 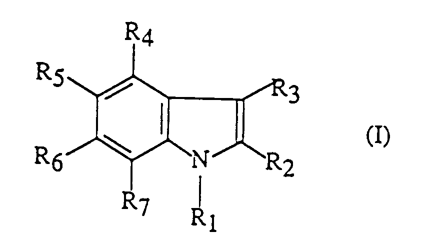 worin gilt:
R₁ und R₃ bedeuten, unabhängig voneinander, ein Wasserstoffatom oder eine C₁₋₄-Alkylgruppe;
R₂ stellt ein Wasserstoffatom, eine C₁₋₄-Alkyl-, Carboxyl- oder eine C₁₋₄-Alkoxycarbonylgruppe dar;
R₄ und R₇ bedeuten, unabhängig voneinander, ein Wasserstoffatom, eine Hydroxy-, C₁₋₄-Alkyl-, Amino-, C₁₋₄-Alkoxy-, C₂₋₄-Acyloxy- oder eine C₂₋₄-Acylaminogruppe;
R₅ bedeutet Wasserstoff, eine Hydroxy-, C₁₋₄-Alkoxy-, C₁₋₄-Alkyl-, Halogen-, Amino-, C₂₋₁₄-Acyloxy-, C₂₋₄-Acylamino- oder eine Trimethylsilyloxygruppe;
R₆ bedeutet Wasserstoff, eine Hydroxy-, C₁₋₄-Alkoxy-, Amino-, C₂₋₄-Acyloxy-, C₂₋₄-Acylamino-, Trimethylsilyloxy- oder eine C₂₋₄-Hydroxyalkylaminogruppe;
R₅ und R₆ können auch, zusammen mit den Kohlenstoffatomen, an die sie gebunden sind, einen Methylendioxy-Ring, der gegebenenfalls mit einer C₁₋₄-Alkyl- oder -Alkoxygruppe substituiert ist, oder einen Carbonyldioxy-Ring bilden; mindestens eine der Gruppen R₄ bis R₇ stellt eine OZ- oder NHR-Gruppierung dar, wobei höchstens eine der Gruppen R₄ bis R₇ NHR bedeutet;
höchstens zwei der Gruppen R₄ bis R₇ bedeuten OZ, wobei, wenn
Z Wasserstoff bedeutet, diese Gruppen in Position 5 und 6 vorliegen;
und wenigstens eine der Gruppen R₄ bis R₇ bedeutet Wasserstoff, wobei, wenn nur eine dieser Gruppen Wasserstoff bedeutet, nur eine Gruppe aus R₄ bis R₇ dann NHR oder OZ und die anderen Gruppen C₁₋₄-Alkylreste bedeuten, wobei R in NHR ein Wasserstoffatom, eine C₂₋₄-Acyl- oder eine C₂₋₄-Hydroxyalkylgruppe und Z in OZ ein Wasserstoffatom, eine C₂₋₁₄-Acyl-, C₁₋₄-Alkyl- oder eine Trimethylsilylgruppe bedeuten
wobei die entsprechenden Salze eingeschlossen sind.

3. Produkt gemäß der Ansprüche 1 oder 2,
dadurch **gekennzeichnet**, daß die Indol-Verbindung aus den Verbindungen ausgewählt ist: 4-Hydroxyindol, 5-Hydroxyindol, 6-Hydroxyindol, 7-Hydroxyindol, 4-Hydroxy-5-methoxyindol, 4-Hydroxy-5-ethoxyindol, 2-Carboxy-5-hydroxyindol, 5-Hydroxy-6-methoxyindol, 6-Hydroxy-7-methoxyindol, 5-Methoxy-6-hydroxyindol, 5,6-Dihydroxyindol, N-Methyl-5,6-dihydroxyindol, 2-Methyl-5,6-dihydroxyindol, 3-Methyl-5,6-dihydroxyindol, 2,3-Dimethyl-5,6-dihydroxyindol, 2-Carboxy-5,6-dihydroxyindol, 4-Hydroxy-5-methylindol, 2-Carboxy-6-hydroxyindol, 6-Hydroxy-N-methylindol, 2-Ethoxycarbonyl-5,6-dihydroxyindol, 4-Hydroxy-7-methoxy-2,3-dimethylindol, 4-Hydroxy-5-ethoxy-N-methylindol, 6-Hydroxy-5-methoxy-2-methylindol, 6-Hydroxy-5-methoxy-2,3-dimethylindol, 6-Hydroxy-2-ethoxycarbonylindol, 7-Hydroxy-3-methylindol, 5-Hydroxy-6-methoxy-2,3-dimethylindol, 5-Hydroxy-3-methylindol, 5-Acetoxy-6-hydroxyindol, 5-Hydroxy-2-ethoxycarbonylindol, 6-Hydroxy-2-carboxy-5-methylindol, 6-Hydroxy-2-ethoxycarbonyl-5-methoxyindol, 6-N-β-Hydroxyethylaminoindol, 4-Aminoindol, 5-Aminoindol, 6-Aminoindol, 7-Aminoindol, N-Methyl-6-β-hydroxyethylaminoindol, 6-Amino-2,3-dimethylindol, 6-Amino-2,3,4,5-tetramethylindol, 6-Amino-2,3,4-trimethylindol, 6-Amino-2,3,5-trimethylindol, 6-Amino-2,3,6-trimethylindol, 5,6-Diacetoxyindol, 5-Methoxy-6-acetoxyindol und aus 5,6-Dimethoxyindol.

4. Produkt gemäß einem der Ansprüche 1 bis 3,
dadurch **gekennzeichnet**, daß die Indol-Verbindung 5,6-Dihydroxyindol, 6-Hydroxyindol, 3-Methyl-5,6-dihydroxyindol, 2-Methyl-5,6-dihydroxyindol, 1-Methyl-5,6-dihydroxyindol, 2-Carboxy-5,6-dihydroxyindol oder deren Mischung ist.

5. Produkt gemäß einem der Ansprüche 1 bis 4,
dadurch **gekennzeichnet**, daß die gefärbten Mineralpartikel aus Eisenoxiden, ausgenommen lamellares Eisenoxid, aus Sulfosilikat-Komplex, Chromoxiden, Ammonium- und Manganpyrophosphaten und aus Eisenferricyanid ausgewählt sind.

6. Produkt gemäß einem der Ansprüche 1 bis 5,
dadurch **gekennzeichnet**, daß die gefärbten Mineralpartikel Abmessungen von 0,01 bis 150 µm aufweisen.

7. Verfahren zur Herstellung eines in jedem der Ansprüche 1 bis 6 definierten Produkts,
dadurch **gekennzeichnet**, daß man in einem wässrigen Milieu eine in den Ansprüchen 2, 3 oder 4 definierte Indol-Verbindung und gefärbte Mineralpartikel einer Größe von weniger als 200 µm vermischt und dann das Melanin-Pigment durch Oxidation der Indol-Verbindung der Formel (I) bildet.

8. Verfahren gemäß Anspruch 7,
dadurch **gekennzeichnet**, daß die Oxidation langsam an der Luft bei alkalischem pH durchgeführt wird.

9. Verfahren gemäß Anspruch 7,
dadurch **gekennzeichnet**, daß die Oxidation mit Sauerstoff der Luft in Gegenwart eines metallhaltigen Katalysators durchgeführt wird.

10. Verfahren gemäß Anspruch 7,
dadurch **gekennzeichnet**, daß die Oxidation durch Zugabe oxidierender Mittel aus Wasserstoffperoxid, organischen Persäuren und deren Persalzen, Perjodsäure und ihren Salzen, Permanganaten, Bichromaten, Natriumhypochlorit, Alkalichloriten, Kaliumferricyanid, Ammoniumpersulfat, Silberoxid, Bleioxid, Eisen(III)chlorid, Natriumnitrit, Salzen seltener Erden und aus organischen oxidierenden Mitteln durchgeführt wird, die aus o- und p-Benzochinonen, o- und p-Benzochinonmonoiminen oder -diiminen, 1,2- und 1,4-Naphthochinonen, 1,2- und 1,4-Naphthochinonmono- oder -diiminen ausgewählt sind.

11. Verfahren gemäß Anspruch 7,
dadurch **gekennzeichnet**, daß die Oxidation mit Wasserstoffperoxid in ammoniakalischem Milieu durchgeführt wird.

12. Verfahren gemäß einem der Ansprüche 7 bis 11,
dadurch **gekennzeichnet**, daß das Reaktionsmilieu ein Milieu ist, das im wesentlichen ein Nicht-Lösungsmittel für die Partikel und ein Lösungsmittel für die Indol-Verbindung der Formel (I) ist, das aus Wasser oder einer Mischung aus Wasser und Lösungsmittel(n) zusammengesetzt ist.

13. Verfahren gemäß einem der Ansprüche 7 bis 12,
dadurch **gekennzeichnet**, daß das Lösungsmittel aus Ethyl-, Propyl- oder Isopropylalkohol, t-Butylalkohol, Alkylenglycolen, Alkylethern von Alkylenglycolen und aus Methyllactat ausgewählt ist.

14. Verfahren gemäß einem der Ansprüche 7 bis 13,
dadurch **gekennzeichnet**, daß die Indol-Verbindung in Gewichtsanteilen von 0,1 bis 10 und vorzugsweise von 0,5 bis 7 Gew.%, bezogen auf das Gesamtgewicht des Reaktionsmilieu, und die gefärbten Mineralpartikel in Mengenanteilen von 0,05 bis 70 Gew.% eingesetzt werden.

15. Kosmetische Zusammensetzung,
dadurch **gekennzeichnet**, daß sie in einem kosmetisch geeigneten Milieu mindestens ein in jedem der Ansprüche 1 bis 6 definiertes oder gemäß jedem der Ansprüche 7 bis 14 hergestelltes Produkt enthält.

16. Zusammensetzung gemäß Anspruch 15,
dadurch **gekennzeichnet**, daß sie in Form einer Lotion, verdickten Lotion, eines Gels, einer Creme, Milch, eines Puders oder Stifts vorliegt und gegebenenfalls als Aerosol in Form eines Spray oder Schaums zubereitet ist.

17. Zusammensetzung gemäß Anspruch 15 oder 16,
dadurch **gekennzeichnet**, daß sie dazu bestimmt ist, zum Schminken der Haut, Haare, Nägel, Wimpern und Augenbrauen verwendet zu werden, und in flüssiger, fester oder pastenartiger, wasserfreier oder wässriger Form vorliegt.

18. Zusammensetzung gemäß einem der Ansprüche 15 bis 17 zum Schutz der menschlichen Epidermis vor den UV-Strahlen der Sonne,
dadurch **gekennzeichnet**, daß sie in Form einer Suspension oder Dispersion in Lösungsmitteln oder Fettkörpern oder in Form von Emulsionen, Pomaden, Gelen, Feststoffstäbchen oder Aerosol-Schäumen vorliegt.

19. Zusammensetzung gemäß einem der Ansprüche 15 bis 18,
dadurch **gekennzeichnet**, daß sie Fettkörper, organische Lösungsmittel, Silikone, Verdickungsmittel, Weichmacher, oberflächenaktive Mittel, Sonnenfilterstoffe, Antischaummittel, hydratisierende Mittel, Parfüm-Produkte und Konservierungsmittel, Antioxidantien, Beaufschlagungsmittel, Sequestriermittel, Behandlungsmittel, Treibmittel, alkalisch oder sauer stellende Mittel oder weitere Pigmente enthält.

20. Zusammensetzung gemäß Anspruch 19,
dadurch **gekennzeichnet**, daß sie Nanopigmente aus Metalloxiden aus Oxiden von Titan, Zink Cer oder Zirkon enthält, wobei diese Nanopigmente einen mittleren Durchmesser von weniger als 100 nm aufweisen und umhüllt sind oder nicht.

21. Zusammensetzung gemäß einem der Ansprüche 15 bis 20,
dadurch **gekennzeichnet**, daß das Pulver in Form gefärbter Mineralpartikel, die das Melanin-Pigment enthalten, in Konzentrationen von 0,05 bis 35 und vorzugsweise von 0,1 bis 20 Gew.% vorliegt, bezogen auf das Gesamtgewicht der Zusammensetzung.

22. Verwendung des in jedem der Ansprüche 1 bis 6 definierten oder gemäß dem in jedem der Ansprüche 7 bis 14 definierten Verfahren hergestellten Produkts zum Schutz der menschlichen Epidermis.

23. Verwendung des in jedem der Ansprüche 1 bis 6 definierten oder gemäß dem in jedem der Ansprüche 7 bis 14 definierten Verfahren hergestellten Produkts in Produkten zum Schminken der Haut, Haare, Nägel, Wimpern oder Augenbrauen.
